(19) Europäisches Patentamt
European Patent Office
Office européen des brevets

(11) Publication number: **0 041 665**
**B1**

(12) # EUROPEAN PATENT SPECIFICATION

(45) Date of publication of patent specification: **21.08.85**

(51) Int. Cl.$^4$: **A 61 K 9/02**, A 61 K 9/36, A 61 K 9/66

(21) Application number: **81104132.6**

(22) Date of filing: **29.05.81**

(54) **Medicament capsules for rectal application.**

(30) Priority: **05.06.80 JP 76264/80**
**24.02.81 JP 26624/81**

(43) Date of publication of application:
**16.12.81 Bulletin 81/50**

(45) Publication of the grant of the patent:
**21.08.85 Bulletin 85/34**

(84) Designated Contracting States:
**AT CH DE FR IT LI NL SE**

(56) References cited:
**EP-A-0 018 605**
**GB-A-2 006 011**
**GB-A-2 006 217**
**US-A-2 580 683**

(73) Proprietor: **SHIONOGI & CO., LTD.**
**12, Dosho-machi 3-chome Higashi-ku**
**Osaka 541 (JP)**

(73) Proprietor: **Shin-Etsu Chemical Co., Ltd.**
**6-1, Ohtemachi 2-chome**
**Chiyoda-ku, Tokyo (JP)**

(72) Inventor: **Takagishi, Yasushi**
**5-22, Horikiri-cho**
**Nishinomiya-shi Hyogo-ken (JP)**
Inventor: **Doi, Yoshio**
**10-10, Wakazono-cho Ibaraki-shi**
**Osaka (JP)**
Inventor: **Aida, Kanji**
**2-9-3, Sakurai Minoo-shi**
**Osaka (JP)**
Inventor: **Hoshi, Noboru**
**9-1, Higashi-Honcho Higashi-Kurume-shi**
**Tokyo (JP)**
Inventor: **Osuga, Kiichiro**
**376-22, Kawara-cho**
**Kusatsu-shi Shiga-ken (JP)**

(74) Representative: **Jaeger, Klaus, Dr.rer.nat. Dipl.-Chem. et al**
**Jaeger, Grams & Pontani Patentanwälte**
**Bergstrasse 48 1/2**
**D-8035 München-Gauting (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 041 665 B1

**Description**

The present invention relates to a novel medicament capsule for rectal application. More particularly, the invention relates to a medicament form of a hard capsule made of a novel enterosoluble cellulose derivative encapsulating a therapeutically effective ingredient capable of being absorbed through the rectum.

As is well known, a variety of medicines are used in a medicament form suitable for rectal application with an object either to obtain a systemic action or to obtain a local action of the effective ingredient. Several of the examples of the medicines of which a systemic action is desired by the rectal application include antipyretic, anodynic and antiphlogistic agents such as aspirin®, aminopyrine, sulpyrin, phenylbutazone, oxyphenbutazone, indomethacin and antispasmodic agents such as butyl scopolamine bromide, antibiotics such as erythromycin, and anti-tuberculosis agents such as ethionamide. Those medicines of which a local action is desired include, on the other hand, astringents, local anesthetics and bactericidal agents with or without admixture of an adrenocortical hormone for haemorrhoids.

At any rate, the effective ingredient in the medicine administrated by rectal application is directly absorbed in the venous plexus of the rectum to be distributed throughout the body by the blood circulation without passing the portal vein and the liver. Therefore, a rectally applicable medicament form is preferable particularly for the medicines of which the effective ingredient causes a disorder in the stomach when orally administrated or the ingredient is susceptible to decomposition in the digestive tract or in the liver resulting in decreased effectiveness of the medicine.

As is well known, the medicament forms for rectal application in general include suppositories and so-called rectal capsules.

Suppositories as a medicament form are prepared usually by dispersing the therapeutically active ingredient in a base such as cacao butter, polyethyleneglycol and mixtures of higher fatty acid glycerides solidifying and shaping the blend in to a desired form, e.g. a conical or cannonball-like form, suitable for insertion into the coelom through the anus. Suppositories are the most widely used medicament form for rectal application and effective by releasing the effective ingredient in the rectum when melted at the body temperature or dissolved in the rectal fluid.

Suppositories present a very convenient means for rectal application of the medicine but not without various problems in the preparation and storage thereof. Several of the problems are that an efficient means is required for the uniform dispersion of the effective ingredient in the highly consistent suppository base, that suppositories are not a suitable medicament form for an ingredient susceptible to thermal decomposition because the ingredient must be distributed in the base molten by heating, that suppositories must be stored in a cool place because deformation of suppositories is unavoidable at a relatively high temperature and that specific facilities are necessary for the preparation thereof to be in compliance with the aforementioned problems.

On the other hand, a rectal capsule is a modification of the suppository in a sense and can be prepared in a manner similar to the preparation of soft capsules. That is, the therapeutically effective ingredient, alone or with admixture of other additives according to need, is shaped by lightly compressing and encapsulated and further shaped with a capsule base such as gelatin. Therefore, preparation of rectal capsules also requires specific facilities and skilful work.

In view of the above problems in the rectal capsules, it is another possible way to utilize an ordinary gelatin-made capsule for oral administration as the medicament form for rectal application. Several difficulties are, however, encountered in the use of gelatin capsules for rectal application. For example, medicines with acidity or in an aqueous form cannot be encapsulated in both hard and soft gelatin-made capsules. Further, powdery medicines encapsulated in a soft capsule are not free from the problem in the stability in addition to the burdensomeness in the preparation of the medicament form.

A further problem in the medicament capsules for rectal application is that the disintegrability of the capsules and the absorbability of the effective ingredients in the rectum are widely diversified among the individuals administrated with the capsules by rectal application.

GB—A—2 006 217 discloses cellulose derivatives which are mixed esters of an alkoxy or hydroxy alkoxy substituted cellulose ether which are useful for the enteric coating of pharmaceutical dosage forms for oral application. The coating liquid is prepared by dissolving the cellulose derivative in a suitable organic solvent, then is applied to the pharmaceutical solid dosage form to be coated with the cellulose derivatives, such as capsules of gelatine, which coating is preformed by use of a conventional coating machine, and the coating then is dried to finally form the enteric coating of the capsule. For rectal application, however, also such a coated gelatine capsule neither is better nor is worse than an ordinary uncoated gelatine capsule as discussed above.

Accordingly, it has been eagerly desired to develop a medicament capsule for rectal application easy to prepare into the medicament form with excellent stability during storage and capable of exhibiting uniform disintegrability in the rectum and the absorbability of the effective ingredient through the rectum.

Thus the object of the present invention is to provide a novel and improved medicament capsule for rectal application with which the problems in the conventional suppositories and rectal capsules as well as the problems involved in the application of ordinary hard or soft capsules to the rectal use are completely solved.

The medicament capsule for rectal application according to the present invention comprises (a) a hard capsule shell made of a mixed ester of a cellulose ester substituted by hydroxy-substituted and/or unsubstituted alkyl groups and esterified with aliphatic monoacyl groups and acidic succinyl groups and (b) a therapeutically effective ingredient capable of being rectally absorbed and encapsulated in the said hard capsule shell.

It has also been established that the uniformity in the absorption of the effective ingredient encapsulated in the medicament capsule for rectal application is greatly improved when the effective ingredient is contained in the capsule shell in a liquid form and the osmotic pressure of the liquid in the capsule is made higher than the osmotic pressure of the rectal fluid.

Hereinunder the invention is described in more detail.

Figure 1 shows the disintegration time of the inventive capsules in the test solutions as a function of the value of pH of the solution.

Figure 2 shows the change in the concentration of sulfamethoxazole in blood plasma of rabbits administrated by rectal application with the medicine in the form of the inventive medicament capsule containing the same as a function of time.

Figures 3 to 6 show the change in the concentration of sulfamethoxazole in blood plasma of rabbits administrated by rectal application with the medicine in the form of the medicament capsule containing the same, of which the osmotic pressure of the content is 196 kPa/kg $H_2O$, as a function of time.

Figure 7 shows the change in the concentration of sulfamethoxazole in blood plasma of rabbits administrated by rectal application with the medicine in the form of the medicament capsule containing the same, of which the osmotic pressure of the content is 5684 kPa/kg $H_2O$, as a function of time.

The hard capsule shell used in the inventive medicament capsule for rectal application is shaped of the above mentioned enteric cellulose derivative and has many advantages, in addition to the excellent enterosolubility, of the intoxicity to the living body and the chemical stability in the lapse of time without the danger of producing any toxic or harmful decomposition products during storage as well as excellent physical stability over a long period of time to retain the mechanical properties such as pliability even without formulation of a plasticizer.

The enteric cellulose derivative for shaping the shell of the inventive medicament capsule is a mixed ester of a cellulose ether substituted or etherified by alkyl groups and/or hydroxyalkyl groups and esterified by aliphatic monoacyl groups and acidic succinyl groups. The preparation of such a mixed ester of cellulose ether is described, for example, in U.S. Patent No. 4,226,981. Several of the advantages obtained with this type of cellulose derivatives are as follows.

(1) The cellulose derivative can form a film having high pliability by use of no or a very small amount of a plasticizer.

(2) The films thus formed do not stick to each other.

(3) The cellulose derivative is chemically and physically stable without deterioration over a long period of time even in a highly humid and hot atmosphere.

(4) The purification process of the esterified products after completion of the reaction can be performed easily so that the purified products of the cellulose derivative contain no undesirable impurities.

As is mentioned above, the mixed ester of the cellulose ether used for shaping the capsule shell has two kinds of esterifying substituent groups; i.e. aliphatic monoacyl groups represented by the general formula R—CO—, in which R is a monovalent aliphatic group or, in particular, an alkyl group, and acidic succinyl groups expressed by the formula $HO—CO—CH_2CH_2—CO—$ bonded to the cellulose ether through ester linkages and obtained by the esterification reaction of a cellulose ether with succinic anhydride and an anhydride of an aliphatic monocarboxylic acid.

The cellulose ether above mentioned has necessarily alkyl groups and/or hydroxyalkyl groups as the substituent groups bonded to the glucose residue through ether linkages and is exemplified by alkylcelluloses such as methylcellulose, ethylcellulose and propylcellulose, hydroxyalkylcelluloses such as hydroxyethylcellulose, hydroxypropylcellulose and hydroxybutylcellulose, and hydroxyalkyl alkylcelluloses such as hydroxyethyl methylcellulose, hydroxyethyl ethylcellulose, hydroxypropyl methylcellulose, hydroxypropyl ethylcellulose, hydroxybutyl methylcellulose and hydroxybutyl ethylcellulose as well as those cellulose ethers having two kinds or more of the hydroxyalkyl groups such as hydroxyethyl hydroxypropylcellulose, hydroxyethyl hydroxybutylcellulose and hydroxyethyl hydroxypropyl methylcellulose.

These cellulose ethers are not particularly limitative in respect of the molecular weight and the degree of substitution by the alkyl groups and/or hydroxyalkyl groups. When the cellulose ether is an alkylcellulose or a hydroxyalkyl alkylcellulose, however, it should be noted that the difficulty in the esterification reaction with the above mentioned acid anhydrides is increased if the degree of substitution by the alkyl groups is excessively large over a limit of, say, 2.5 alkyl groups per glucose residue.

The anhydride of the aliphatic monocarboxylic acid used in the esterification of the cellulose ether in combination with succinic anhydride is exemplified by the anhydrides of acetic, propionic, butyric and valeric acids. These acid anhydrides as well as succinic anhydride may not be of special grades and commercially available ones can be used without further purification.

The esterification reaction of the cellulose ether with the acid anhydrides is carried out in several different ways. For example, the cellulose ether and the acid anhydrides are reacted in a carboxylic acid such as acetic acid, propionic acid and butyric acid as the reaction medium in the presence of an alkali metal salt of a carboxylic acid such as sodium acetate or potassium acetate as a catalyst. Alternatively, the reaction is carried out in a suitable organic solvent such as dimethylformamide or acetate in the presence of a basic catalyst such as α-picoline or pyridine.

The average degrees of substitution per glucose unit by the acidic succinyl groups and the aliphatic acyl groups are determined in accordance with the desired performance of the mixed ester product as well as the type of the cellulose ether as the starting material. Generally speaking, the average numbers of the substituent groups are desirably at least 0.1 and at least 0.05 per glucose unit for the acidic succinyl groups and the aliphatic monoacyl groups, respectively, since smaller degrees of substitution result in unsatisfactory pliability and enteric solubility of the cellulose derivative.

The shaping procedure of the capsule shells from the above described cellulose derivative is rather conventional and may be performed by the dipping method using molding pins. Thus, the cellulose derivative is dissolved in a suitable solvent which is an organic solvent or a mixture of an organic solvent and water into a dipping solution of an adequate concentration and, after defoaming, if necessary, a molding pin is dipped therein and then pulled up gradually so as that the viscous film of the solution adhering to the molding pin is dried and converted to a solid film of the form of a capsule shell, which is detached from the molding pin followed, if necessary, by finishing to give a capsule shell of the desired size and form.

The organic solvent used for the preparation of the above mentioned dipping solution is exemplified by methyl aclohol, ethyl alcohol, acetone, ethyleneglycol monomethyl ether, ethyleneglycol monoethyl ether and ethyl acetate, which may be used either alone or in combination of two kinds or more. The concentration of the dipping solution is of course determined in consideration of the workability in the shaping procedure by the dipping method as well as the desired wall thickness of the finished capsule shells. Usually, capsule shells of a smaller wall thickness are prepared by use of a less concentrated solution and the concentration should be increased when capsule shells of a larger wall thickness are to be prepared. The wall thickness of the capsule shells is usually in the range from 100 to 150 μm or, preferably, from 100 to 120 μm in consideration of the balance of the bioavailability of the effective ingredient therein and the physical properties of the capsules.

The dipping solution may be formulated with admixture of several conventional additives according to need such as coloring agents, taste- and flavor-improvers, flavors or plasticizers. Needless to say, the amounts of these optional additives to be added to the dipping solution are limited not to adversely affect the advantageous properties inherent to the mixed ester of the cellulose derivative. In addition to the above described dipping method for shaping the capsule shells, any other conventional shaping methods can be used such as compression molding under heating.

In the following, the preparation of the cellulose derivatives for shaping the capsule shells used in the inventive rectally applicable medicament capsules is described in detail by way of examples along with the characterization of the cellulose derivatives and the capsule shells. In the following, parts are all given by parts by weight.

Preparation 1

Into a reaction vessel equipped with a stirrer were introduced 100 parts of glacial acetic acid and 20 parts of sodium acetate together with a cellulose ether, succinic anhydride and an anhydride of an aliphatic monocarboxylic acid of the kinds and in amounts as indicated in Table 1 below and the esterification reaction was conducted by agitating the reaction mixture for 3 hours at 85°C. Then, water was added to the reaction mixture to precipitate the reaction product which was taken by filtration, washed with water and dried to give a mixed ester of the cellulose ether containing acidic succinyl groups and aliphatic monoacyl groups. The degrees of substitution, D.S., of these ester groups were as summarized in Table 1.

The cellulose ether compounds as the starting materials used in this preparation and denoted by abridgements in Table 1 were as follows.

HPC : hydroxypropylcellulose, the average number of substitution with hydroxypropoxyl groups per glucose unit 3.0

HPMC : hydroxypropyl methylcellulose, the average numbers of substitution with hydroxypropoxyl groups and methoxy groups per glucose unit 0.27 and 1.82, respectively

HEHPC : hydroxyethyl hydroxypropylcellulose, the average numbers of substitution with hydroxyethoxy groups and hydroxypropoxyl groups per glucose unit 2.5 and 0.32, respectively.

TABLE 1

| Sample No. | Starting materials | | | | | Product | |
|---|---|---|---|---|---|---|---|
| | Cellulose ether | | Succinic anhydride, parts | Monocarboxylic acid anhydride | | D.S. of acidic succinyl groups | D.S. of aliphatic monoacyl groups |
| | Compound | parts | | Compound | parts | | |
| 1 | HPC | 20 | 4 | Acetic Anhydride | 20 | 0.20 | 0.86 |
| 2 | HPMC | 20 | 6 | Acetic anhydride | 32 | 0.25 | 0.57 |
| 3 | HEHPC | 20 | 6 | Propionic anhydride | 40 | 0.30 | 0.85 |

In the next place, the above prepared three samples were examined for the stability against hydrolysis and the elongation of the films shaped therefrom along with two kinds of comparative samples No. 4 which was a cellulose acetate phthalate, CAP, having the average degrees of substitution with acetyl groups and phthaloyl groups per glucose unit of 1.84 and 0.76, respectively, and No. 5 which was a hydroxypropyl methylcellulose phthalate, HPMCP, having the average degrees of substitution with hydroxypropoxyl groups, methoxy groups and pthaloyl groups per glucose unit of 0.22, 0.72 and 0.68, respectively. The results are shown in Table 2 below.

Test procedures for the stability against hydrolysis:

a) Determination of free aliphatic monocarboxylic acid

The cellulose derivative kept standing for 6 days or 12 days in atmospheric conditions of 100 % relative humidity at 60°C was subjected to extraction with diethyl ether for 5 hours in a Soxhlet extractor and the aliphatic monocarboxylic acid contained in the ether extract was determined by the gas chromatography

b) Determination of free acids other than the aliphatic monocarboxylic acid

The cellulose derivative kept standing in the same atmospheric conditions as above was dissolved in a 1:1 by volume mixed solvent of methylene chloride and methyl alcohol and further admixed with n-hexane. The solution was then extracted with water and the overall amount of the free acids in the water extract was determined by titration with a 0.1 N aqueous solution of sodium hydroxide and the amount of the free acids other than the aliphatic monocarboxylic acid was obtained by the difference between this value and the amount of the free aliphatic monocarboxylic acid obtained in a) above.

Measurement of the elongation of the films:

Films were prepared by the casting method from a solution of the cellulose derivative dissolved in a 1:1 by volume mixed solvent of methylene chloride and methyl alcohol. Measurement of the elongation was performed on an automatic recording tensile tester at 25°C.

TABLE 2

| Sample No. | Stability against hydrolysis, % by weight of free acid | | | | Elongation of film % |
|---|---|---|---|---|---|
| | After 6 days | | After 12 days | | |
| | (a) | (b) | (a) | (b) | |
| 1 | 0.3 | 0.1 | 0.6 | 0.2 | 20 |
| 2 | 0.5 | 0.4 | 0.7 | 0.5 | 10 |
| 3 | 0.7 | 0.4 | 0.6 | 0.4 | 10 |
| 4 | 7.0 | 9.3 | 11.0 | 13.2 | 4 |
| 5 | — | 3.1 | — | 3.5 | 3 |

(a): free aliphatic monocarboxylic acid (see test procedure a)
(b): free acids other than aliphatic monocarboxylic acid (see test procedure b)).

Preparation 2

Following is a description of the preparation of hard capsule shells shaped of the above described mixed esters of the cellulose derivatives as the base material. The capsules were subjected to the test of the disintegrability to give the results shown in Table 3 below.

A viscous dipping solution was prepared by dissolving 90 g of the Sample No. 2 prepared in Preparation 1 above in 210 g of a 6:4 by volume mixed solvent of acetone and ethyl alcohol followed by defoaming by standing at room temperature.

A molding pin either for capsule cap or for capsule body of the size No. 0 after mold-release treatment was dipped in the above prepared dipping solution and then gradually pulled up from the solution to form a film of the viscous solution adhering around the molding pin. Complete drying of the liquid film around the molding pin at 40 to 42°C gave a capsule shell half having a wall thickness of 150 µm which was demounted from the molding pin and finished to a clear capsule shell half rich in pliability.

A capsule made by coupling the above prepared capsule cap and body was filled with lactose powder and sealed around the coupling portion with the same viscous solution of the cellulose derivative. The disintegrability of the thus prepared capsules was tested in the first and the second solutions having a pH of 1.2 or 7.5, respectively, according to the Ninth Revised Japanese Pharmacopoeia or the McIlvaine buffer solutions (mixtures of disodium hydrogen phosphate and citric acid) having a pH of 4.5, 5.0, 5.5 or 6.0. The dissolvability behavior of the capsules in each of the test solutions was as follows.

i) No noticeable changes within 2 hours in the first solution of pharmacopoeia at pH 1.2.
ii) No noticeable changes within 2 hours in the McIlvaine buffer solution at pH 4.5.
iii) No noticeable changes within 2 hours in the McIlvaine buffer solution at pH 5.0.
iv) The capsule was dissolved and broken to release the content after 20 to 25 minutes in the McIlvaine buffer solution at pH 5.5.
v) The capsule was dissolved and broken to release the content after 8 to 10 minutes in the McIlvaine buffer solution at pH 6.0.
vi) The capsule was dissolved and broken to release the content after 6 to 9 minutes in the second solution of pharmacopoeia a pH 7.5.

Preparation 3

Capsule shells having a wall thickness of 150 µm were prepared in the same manner as in Preparation 2 above except that the dipping solution used for pin molding was a solution of 20 g of the Sample No. 1 prepared in Preparation 1 in 80 g of a 8:2 by volume mixed solvent of ethyl alcohol and water. The capsules were also clear and rich in pliability.

The dissolvability test of the capsules was conducted in the same manner as above with first and the second solutions according to the pharmacopoeia to give the results as follows.

vii) No noticeable changes within 2 hours in the first solution at pH 1.2.
viii) The capsule was dissolved and broken to release the content after 15 to 20 minutes in the second solution at pH 7.5.

Preparation 4

Capsule shells having a wall thickness of 150 µm were prepared in the same manner as in Preparation 2 above except that the viscous dipping solution used for pin molding was a solution of 25 g of the Sample No. 3 prepared in Preparation 1 in 75 g of a 1:1 by volume mixed solvent of acetone and ethyleneglycol monomethyl ether. The capsules were also clear and rich in pliability.

The dissolvability test of the capsules was conducted in the same manner as above with the first and the second solutions according to the pharmacopoeia to give the results as follows.

ix) No noticeable changes within 2 hours in the first solution at pH 1.2.

x) The capsule was dissolved and broken to release the content after 15 to 25 minutes in the second solution at pH 7.5.

Preparation 5

Capsule shells of No. 0 size were prepared with a hydroxypropyl methylcellulose acetate succinate in a simillar manner to Preparation 2 in two different wall thicknesses of (A) 150 μm and (B) 100 μm each weighing 125 mg or 110 mg, respectively, for the capsule cap and body coupled together.

The dissolvability behavior of each of the above prepared capsules filled with barium sulfate was examined in a test apparatus for disintegration specified in the pharmacopoeia but without the auxiliary plastic disk to give the results graphically illustrated in Figure 1.

As is clear from the figure, the capsules were stable at pH 5.0 for 2 hours or longer irrespective of the wall thickness.

The dissolution of the capsules took place only at a pH of 5.5 or higher and was considerably rapid at a pH of 6.0 or higher with somewhat larger velocities for the capsules of smaller wall thickness than for those with larger wall thickness.

Simulating in vivo test

Simulating capsules were prepared by filling the comparative capsules A and B prepared in Preparation 5 above with a 30 % by weight suspension of barium sulfate in water or in sesame oil as a contrast medium. The capsules were inserted each into the rectum of a male rabbit having a body weight of about 3 kg after a fasting period of 17 hours about 3 cm deep from the anus and traced roentgenographically with soft X-rays by use of an apparatus Model Softex CMEW-2, manufactured by Nippon Softex Co., Japan, to examine the in vivo dissolvability behavior of the capsules. The results of the average time in minutes for the dissolution of the capsules in the rabbit recta were as shown in Table 3 below.

TABLE 3

| Content of capsule | Capsule A | Capsule B |
|---|---|---|
| Barium sulfate/water suspension | 40 minutes | 20 minutes |
| Barium sulfate/sesame oil suspension | 95 minutes | 50 minutes |

The time to the dissolution of the capsule A was about twice as long as that with the capsule B in parallel with the general trend in the in vitro test described in Preparation 5 and illustrated in Figure 1. The aqueous suspension filling the capsule apparently accelerated the dissolution of the capsule in comparison with the suspension in sesame oil.

As is understood from the above given description of the preparations and the simulating in vivo test, the medicament capsules according to the present invention are very suitable as a dosage form for rectal application. It may be concluded from the above results that (a) the wall thickness of the capsule in the invention can be the same as or slightly smaller than in the ordinary capsules for oral administration provided no troubles are caused in rectal application and (b) the form of the capsule is desirably conical or cannonball-like in order to facilitate rectal application but the conventional capsule assemblage of cap and body coupled together having dome-like or semispherical closed ends are well suitable for the purpose.

The medicines to fill the inventive medicament capsules are not limited to particular ones and may include all kinds of medicines hitherto formulated in suppositories as the therapeutically effective ingredient such as antipyretics, anodynes, antiphlogistics, antispasmodics, antibiotics, antituberculosis agents, astringents, local anesthetics, bactericides and laxatives.

As is understood from the foregoing, the inventive medicament capsules are practically very advantageous because the dosage form preparation is very easy and no special care is needed in the storage thereof by virtue of the utilization of a hard capsule made of a specific material having excellent stability. These advantages will be more clearly understood when comparisons are made between the characteristics of the inventive medicament capsules and the conventional suppositories and gelatin-made hard and soft capsules as tabulated below in Table 4.

TABLE 4

| Dosage form for rectal application | | Inventive medicament capsule | Suppository | Gelatin-made soft capsule | Gelatin-made hard capsule |
|---|---|---|---|---|---|
| Temperature condition in storage | | Room temperature | Cool place | Room temperature | Room temperature |
| Stability of powdery medicine | | Good | Fair | Fair | Good |
| Applicability of liquid medicine | Aqueous | Yes | No | No | No |
| | Oily | Yes | Yes | Yes | Yes |
| Filling work with the medicine | | Manual or machine work | Machine work | Machine work | Manual or machine work |
| Applicability of acidic medicine | | Yes | Yes | No | No |

Dose test 1

In this dose test and hereunder, medicament capsules were prepared of the capsule shells having a wall thickness of 100 μm obtained in Preparation 5. Medicament capsules were prepared by filling the capsules with a suspension of sulfamethoxazole as the test ingredient in water or in sesame oil in such an amount that each capsule contained 240 mg of the effective ingredient. In parallel, suppositories were prepared as a dosage form for control by casting a uniform blend of sulfamethoxazole in a molten suppository base at 50°C, which was a mixture of mono-, di- and triglycerides of saturated fatty acids having from 12 to 18 carbon atoms in a molecule (Witepsol®, a product by Nynamit Nobel Co.) into a conventional suppository form. Each piece of the suppositories also contained 240 mg of the effective ingredient. Each a piece of the above test capsules and the control suppositories was composed of the respective materials in amounts indicated in Table 5 in mg.

TABLE 5

| | Test capsule B-1 | Test capsule B-2 | Control suppository |
|---|---|---|---|
| Sulfamethoxazole | 240 mg | 240 mg | 240 mg |
| Water | 500 | — | — |
| Sesame oil | — | 390 | — |
| Capsule | 110 | 110 | — |
| Witepsol | — | — | 760 |
| Total | 850 | 740 | 1000 |

Each of the above prepared capsules and suppositories was inserted to the rectum about 3 cm deep from the anus of a male rabbit having a body weight of 2.9 to 3.1 kg after a fasting period of 17 hours and blood was taken periodically from the vein on the earlobe. Blood plasma was obtained from the blood by centrifugal separation and the concentration of sulfamethoxazole in the plasma was determined to give the results as plotted in Figure 2.

As is clear from the results, the inventive capsule containing the aqueous suspension of the medicine gave a larger velocity of increase in the concentration of the medicine in the blood plasma as well as a higher maximum concentration than with the Witepsol-based suppository. These results support the practical advantages of the rectally applicable medicament capsules of the present invention over the conventional suppository as a dosage form. When the aqueous suspension contained in the capsule was replaced with a suspension in sesame oil, retardation was noted in the rate of absorption of the effective ingredient though not to an extent of impracticality.

Dose test 2

A pasty mixture was prepared by uniformly blending 50 g of sulfamethoxazole and 30 g of olive oil and a capsule shell having a wall thickness of 100 µm obtained in Preparation 5 was filled with 800 mg of the paste containing 500 mg of the effective ingredient to give a rectally applicable medicament capsule which was subjected to the animal test in the same manner as in Dose Test 1 above to give equally good results.

Dose test 3

A pasty mixture was prepared by uniformly blending 22.5 g of sulfamethoxazole and 36 g of olive oil and a capsule shell of No. 2 size having a wall thickness of 100 µm prepared in the same manner as in Preparation 5 was filled with 390 mg of the paste containing 150 mg of the effective ingredient to give a rectally applicable medicament capsule which was subjected to the animal test in the same manner as in Dose Test 1 to give equally good results.

Dose test 4

An oily suspension was prepared by uniformly blending 75 g of aspirin, 141 g of olive oil and further 7.8 g of a finely divided silicic anhydride and a capsule shell obtained in Preparation 5 was filled with 746 mg of the suspension containing 250 mg of aspirin to give a rectally applicable medicament capsule which was subjected to the animal test in a manner similar to Dose Test 1 above to give equally good results.

Dose test 5

An oily suspension was prepared by uniformly blending 50 mg of betamethasone (9-fluoro-11β,17,21-trihydroxy-16β-methylpregna-1,4-diene-3,20-dione), 63.6 g of sesame oil and further 2.6 g of a finely divided silicic anhydride and a capsule shell of No. 0 size obtained in Preparation 5 was filled with 663 mg of the suspension containing 0.5 mg of the effective ingredient to give a rectally applicable medicament capsule which was subjected to the animal test in a manner similar to Dose Test 1 above to give equally good results.

As is understood from the above description and the results of the dose test, the medicament capsules according to the present invention are very advantageous over the conventional dosage forms for rectal application in that a wide versatility is obtained in the form of the medicines to be contained in the capsule shells including any kind of aqueous liquid medicines such as aqueous solutions, aqueous suspensions, emulsions and the like which hitherto have been considered to be quite unsuitable for rectal application.

In further continuing their investigations directed to the improvement of the rectally applicable medicament capsules, the inventors have become aware of the fact that, when a capsule containing either an aqueous or an oleic medicine is rectally applied to a test animal such as a rabbit, the disintegrability of the capsule in the rectum and the absorbability of the effective ingredient through the rectum are widely diversified from individual to individual to an unimaginable extent.

To demonstrate the above mentioned diversity in the effectiveness or bioavailability of the rectally applied medicines among individual test animals, dose tests similar to the Dose Test 1 described above were undertaken with several rabbits with sulfamethoxazole as the test ingredient.

Thus, several medicament capsules were prepared by use of the capsule shells obtained in Preparation 5 above, each capsule being filled with an aqueous solution of 70 mg of sulfamethoxazole in 600 mg of a 0.001 N hydrochloric acid containing 2 % by weight of a hydroxypropylcellulose. For comparison, suppositories were prepared, each being composed of 70 mg of sulfamethoxazole and 930 mg of Witepsol® as the suppository base.

Each one of the above prepared medicament capsules or the suppositories was inserted to the rectum about 3 cm deep from the anus of one of the male rabbits No. 1 to No. 7 having a body weight of 2.9 to 3.1 kg after a fasting period of 17 hours and the concentration of the effective ingredient in the blood plasma was periodically determined in the same manner as in Dose Test 1. The results are shown in Figures 3 to 6, of which Figures 3 to 5 give a comparison between the medicament capsule and the suppository in the rabbits No. 1, No. 2 and No. 3, respectively, the solid lines being for the capsules and the broken lines being for the suppositories and Figure 6 gives the results with the capsules in the rabbits No. 4 to No. 7 by the curves IV to VII, respectively.

As is shown in Figure 3, the rates of absorption for the capsule and the suppository were about the same in the rabbit No. 1 while the rates were widely different in the rabbits No. 2 and No. 3 as is clear from Figures 4 and 5. Figure 6 more clearly indicates the dependency of the absorbability of a medicine on individuals in the rectal application of medicament capsules. Although differences among individuals may be not surprising since the disintegration of the capsule and the absorption of the effective ingredient should be influenced by the amount of the rectal fluid and the value of pH thereof, the extraordinarily large difference, for example, between the rabbit No. 1 and the rabbit No. 2, No. 3 or No. 7 had been quite out of imagination before the tests.

As a result of the investigations directed to solve the above described problem of non-uniformity of the absorption of effective ingredients contained in the rectally applied medicament capsules among individuals, a conclusion has been arrived at that the osmotic pressure of the aqueous liquid medicine contained in the capsule plays a very important role in the disintegration of a rectally applied capsule in a living body and, consequently, in the release of the effective ingredient contained in the capsule.

Further investigations undertaken by the inventors have established that the osmotic pressure of the liquid medicine contained in the capsule should be substantially higher than that of the body fluid or, in particular, the rectal fluid so that the disintegration of the capsule is uniformly accelerated without being affected by the fluctuating living body conditions since the rectal fluid is positively taken into the capsule through the capsule walls and, as a consequence, the difference in the absorbability of the effective ingredient through the rectum is minimized among individuals to overcome the above described serious problem encountered in the dose tests with rabbits as the test animals.

It is known that the osmotic pressure of human body fluids or, in particular, the rectal fluid is usually in the range from 2744 to 2940 kPa/kg $H_2O$. Therefore, the osmotic pressure of the liquid medicine contained in the rectally applicable medicament capsule should be kept substantially higher than above. It should be noted, however, a liquid medicine having an excessively high osmotic pressure may cause diarrhoea when administrated rectally so that the osmotic pressure should be controlled within a range from 3430 to 19600 kPa/kg $H_2O$ or, preferably, in the range from 4900 to 15680 kPa/kg $H_2O$. Meanwhile, the osmotic pressure of the content of the rectally applied capsules in the above described dose tests exhibited in Figures 3 to 6 was about 196 kPa/kg $H_2O$.

The osmotic pressure of the liquid content can be freely controlled by adding a suitable amount of an osmosis adjustment agent to the content of the capsule. Such an osmosis adjustment agent must satisfy several requirements (a) that the capsule shell is not affected thereby, (b) that the liquid content can be made sufficiently hypertonic by the addition of a small amount thereof, and (c) that it is not irritative to the mucous membrane of the rectum not to cause diarrhoea. Therefore, the osmosis adjustment agent should be selected with consideration of these requirements. Several of the examples of preferred osmosis adjustment agents are inorganic salts such as sodium chloride, potassium chloride or ammonium chloride and organic compounds such as glucose, fructose, sorbitol, xylitol, mannitol, glycerin or low-molecular dextran.

The amount of the osmosis adjustment agent to be added to the liquid content of the capsule is usually in the range from 1 to 20 % by weight in the case of the inorganic salts above mentioned and from 5 to 30 % by weight in the case of the above mentioned organic compounds. The amount should be sufficient to make the liquid content of the capsule more hypertonic than the rectal fluid while an excessive amount of the osmosis adjustment agent is undesirable in several respects with a too high osmotic pressure of the liquid content of the capsule in addition to the difficulty in introducing the agent into the capsule.

It should be noted that there are cases where the amount of addition of the osmosis adjustment agent is limited by the solubility thereof even within the above mentioned ranges. An auxiliary means in such a case is the replacement of a part of the above mentioned osmosis adjustment agent with different kinds of additives such as a salt of an organic acid, e.g. potassium citrate, potassium acetate or potassium gluconate in an amount of 1 to 10 % by weight and/or an acidic amino acid or a derivative thereof, e.g. glutamin, glutamic acid, asparagine or aspartic acid in an amount of 0.1 to 1 % by weight.

In the inventive medicament capsules, the pH value of the content of the capsule should be kept in the range from 3 to 4 in order to ensure stability of the capsule shells so that it is sometimes undertaken to add a small amount of hydrochloric acid to the content of the capsule according to need. When the pH is too high above 4, the stability of the capsule shell formed of the enteric cellulose derivative is decreased while an excessively acidic content is undesirable due to the increased irritation to the mucous membrane of the rectum.

Further, the liquid content of the inventive medicament capsule may be admixed with various kinds of additives according to need depending on the properties of the content which may be an aqueous solution, suspension in water or in oil or aqueous emulsion. For example, carboxymethylcellulose, hydroxypropylcellulose, polyvinyl pyrrolidone, propyleneglycol, gum arabic and gum tragacanth as well as fatty acid esters of sucrose, polyoxyethylene castor oil derivatives or bile acid and salts thereof may be added either alone in combination of two kinds or more as a thickening agent, dispersing agent or suspending agent in accordance with the particular object.

The above described control of the osmotic pressure of the liquid content of the inventive medicament capsule for rectal application is very effective in that, with the liquid content of the capsule kept more hypertonic than the rectal fluid, the disintegration of the capsule is accelerated with minimum influence of the variation factors in the living body so that the release velocity of the effective ingredients in the capsule is increased uniformly among the individuals resulting in the enhancement of the bioavailability of the medicine with very much decreased variation among the individual patients.

Following are the results of the experiments undertaken with an object to demonstrate the effectiveness of the osmotic pressure control with rabbits as the test animals.

Dose test 6

Medicament capsules were prepared with the hard capsule shells obtained in Preparation 5, each capsule containing 70 mg of sulfamethoxazole, 12 mg of sodium chloride and 588 mg of a solution containing 2 % by weight of a hydroxypropylcellulose in a 0.001 N hydrochloric acid. The osmotic pressure of the liquid content of the capsules was 5684 kPa/kg $H_2O$. These capsules were applied to several rabbits rectally and the concentration of the effective ingredient in their blood plasma was followed over a length of time in the same manner as before to give the results plotted in Figure 7.

As is clear from this figure, the absorption of the effective ingredient was remarkably improved in the test rabbits No. 2 and No. 3 which showed very poor absorption in the comparative tests shown in Figures 4 and 5, respectively. This effect was especially remarkable in the rabbit No. 3. Generally speaking, the absorbability of the effective ingredient administered with the inventive capsules with a controlled osmotic pressure was about the same level as in the administration in the form of suppositories. It was noted that the absorption of the effective ingredient in the rabbits No. 4 to No. 7 was also improved in comparison with the results shown in Figure 6 with less variation among the rabbits.

No adverse side effects such as irritation and diarrhoea were observed in the irritation test for the rabbit rectum by the addition of sodium chloride to the capsule content.

Dose test 7

Medicament capsules were prepared in the same manner as above, each capsule containing 70 mg of sulfamethoxazole, 2 mg of gum tragacanth, 125 mg of glucose and 473 mg of 0.001 N hydrochloric acid. The osmotic pressure of the liquid content of the capsules was 13230 kPa/kg $H_2O$.

The capsule were rectally applied to several rabbits and the absorption of the effective ingredient was examined by the concentration of it in the blood plasma in the same manner as in Dose Test 6. The absorption of the effective ingredient was apparently improved by the addition of glucose as an osmosis adjustment agent.

In the following Dose Tests 8 to 14, only the formulation of the content of the capsule and the osmotic pressure of the liquid content in each of the tests are given here, although the tests were undertaken by preparing the medicament capsules in the same manner as in Dose Test 6 and the results of the absorption of the effective ingredient were as good as in the preceding Dose Tests.

Dose test 8

| | |
|---|---|
| Sulfamethoxazole | 70 mg/capsule |
| Sodium chloride | 30 |
| Glutamic acid | 6 |
| Propyleneglycol | 6 |
| Distilled water | 558 |
| Total | 670 |
| Osmotic pressure | 15386 kPa/kg $H_2O$ |

Dose test 9

| | |
|---|---|
| Chlorpromazine hydrochloride (tranquilizer) | 10 mg/capsule |
| Ascorbic acid | 1 |
| 5% Sodium chloride solution in 0.001 N hydrochloric acid | 599 |
| Total | 610 |
| Osmotic pressure | 15582 kPa/kg $H_2O$ |

Dose test 10

| | |
|---|---|
| Dicethianime hydrochloride (vitamin) | 50 mg/capsule |
| 5% Sodium chloride solution in 0.001 N hydrochloric acid | 600 |
| Total | 650 |
| Osmotic pressure | 15680 kPa/kg $H_2O$ |

Dose test 11

| | | |
|---|---|---|
| Vinblastine sulfate (anti-tumor agent) | 5 mg/capsule | |
| Sodium laurylsulfate | 5 | |
| 5% Sodium chloride solution in 0.001 N hydrochloric acid | 600 | |
| Total | 610 | |
| Osmotic pressure | 15778 kPa/kg $H_2O$ | |

Dose test 12

| | |
|---|---|
| Acetaminophenone (antipyretic and anodynic agent) | 100 mg/capsule |
| Glucose | 120 |
| Glutamic acid | 5 |
| Distilled water | 400 |
| Total | 625 |
| Osmotic pressure | 13524 kPa/kg $H_2O$ |

Dose test 13

| | |
|---|---|
| Dextromethorphan (antitussive agent) | 2 mg/capsule |
| Glutamic acid | 5 |
| 5% Aqueous sodium chloride solution | 598 |
| Total | 605 |
| Osmotic pressure | 15680 kPa/kg $H_2O$ |

Dose test 14

| | |
|---|---|
| Chlorpheniramine maleate (antihistaminic agent) | 10 mg/capsule |
| Glucose | 130 |
| 0.001 N Hydrochloric acid | 500 |
| Total | 640 |
| Osmotic pressure | 14308 kPa/kg $H_2O$ |

**Claims for the Contracting States CH, DE, FR, IT, LI, NL, SE**

1. A medicament capsule for rectal application which comprises
(a) a hard capsule shell made of a mixed ester of a cellulose ether selected from the group consisting of alkylcelluloses, hydroxyalkylcelluloses and hydroxyalkyl alkylcelluloses and esterified with aliphatic monoacyl groups and acidic succinyl groups, and
(b) a therapeutically effective ingredient capable of being rectally absorbed and encapsulated in the said hard capsule shell.
2. The medicament capsule for rectal application as claimed in claim 1 wherein the alkylcellulose is selected from the group consisting of methylcellulose, ethylcellulose and propylcellulose.
3. The medicament capsule for rectal application as claimed in claim 1 wherein the hydroyalkylcellulose is selected from the group consisting of hydroxyethylcellulose, hydroxypropylcellulose, hydroxyethyl hydroxypropylcellulose and hydroxyethyl hydroxybutylcellulose.

4. The medicament capsule for rectal application as claimed in claim 1 wherein the hydroxyalkyl alkylcellulose is selected from the group consisting of hydroxyethyl methylcellulose, hydroxyethyl ethylcellulose, hydroxypropyl methylcellulose, hydroxypropyl ethylcellulose, hydroxybutyl methylcellulose, hydroxybutyl ethylcellulose and hydroxyethyl hydroxypropyl methylcellulose.

5. The medicament capsule for rectal application as claimed in claim 1 wherein the aliphatic monoacyl group is selected from the group consisting of acetyl, propionyl, butyryl and valeryl groups.

6. The medicament capsule for rectal application as claimed in claim 1 wherein the degrees of substitution with the aliphatic monoacyl groups and acidic succinyl groups in the mixed ester of the cellulose ether are at least 0.05 and at least 0.1, respectively, per glucose unit.

7. The medicament capsule for rectal application as claimed in claim 1 wherein the hard capsule shell has a wall thickness in the range from 100 to 150 μm.

8. The medicament capsule for rectal application as claimed in claim 1 wherein the therapeutically effective ingredient encapsulated in the hard capsule shell is in the form of a liquid medicine having an osmotic pressure substantially higher than the osmotic pressure of the rectal fluid.

9. The medicament capsule for rectal application as claimed in claim 8 wherein the osmotic pressure substantially higher than the osmotic pressure of the rectal fluid is in the range from 3430 to 19600 kPa/kg $H_2O$.

10. The medicament capsule for rectal application as claimed in claim 8 wherein the therapeutically effective ingredient encapsulated in the hard capsule shell in the form of a liquid medicine has a value of pH in the range from 3 to 4.

**Claims for the Contracting State AT**

1. A method of making a medicament capsule for rectal application which comprises

(a) a hard capsule shell made of a mixed ester of a cellulose ether selected from the group consisting of alkylcelluloses, hydroxyalkylcelluloses and hydroxyalkyl alkylcelluloses and esterified with aliphatic monoacyl groups and acidic succinyl groups, and

(b) a therapeutically effective ingredient capable of being rectally absorbed and encapsulated in the said hard capsule shell by first forming said hard capsule from said mixed ester and then filling and closing the pre-formed capsule.

2. The method as claimed in claim 1 wherein the alkylcellulose is selected from the group consisting of methylcellulose, ethylcellulose and propylcellulose.

3. The method as claimed in claim 1 wherein the hydroxyalkylcellulose is selected from the group consisting of hydroxyethylcellulose, hydroxypropylcellulose, hydroxyethyl hydroxypropylcellulose and hydroxyethyl hydroxybutylcellulose.

4. The method as claimed in claim 1 wherein the hydroxyalkyl alkylcellulose is selected from the group consisting of hydroxyethyl methylcellulose, hydroxyethyl ethylcellulose, hydroxypropyl methylcellulose, hydroxypropyl ethylcellulose, hydroxybutyl methylcellulose, hydroxybutyl ethylcellulose and hydroxyethyl hydroxypropyl methylcellulose.

5. The method as claimed in claim 1 wherein the aliphatic monoacyl group is selected from the group consisting of acetyl, propionyl, butyryl and valeryl groups.

6. The method as claimed in claim 1 wherein the degrees of substitution with the aliphatic monoacyl groups and acidic succinyl groups in the mixed ester of the cellulose ether are at least 0.05 and at least 0.1, respectively, per glucose unit.

7. The method as claimed in claim 1 wherein the hard capsule shell has a wall thickness in the range from 100 to 150 μm.

8. The method as claimed in claim 1 wherein the therapeutically effective ingredient encapsulated in the hard capsule shell is in the form of a liquid medicine having an osmotic pressure substantially higher than the osmotic pressure of the rectal fluid.

9. The method as claimed in claim 8 wherein the osmotic pressure substantially higher than the osmotic pressure of the rectal fluid is in the range from 3430 to 19600 kPa/kg $H_2O$.

10. The method as claimed in claim 8 wherein the therapeutically effective ingredient encapsulated in the hard capsule shell in the form of a liquid medicine has a value of pH in the range from 3 to 4.

**Revendications pour les Etats Contractants CH, DE, FR, IT, LI, NL et SE**

1. Capsule médicanale pour application rectale comprenant:

a) un enrobage dur pour capsules, réalisé en un ester mixte d'un éther de cellulose, choisi dans le groupe des alkylcelluloses, hydroxyalkylcelluloses et hydroxyalkylalkylcelluloses, et estérifié par des groupes mono-acyle aliphatiques et par des groupes succinyle acides, et

b) un ingrédient thérapeutiquement actif susceptible d'être absorbé par voie rectale et enrobé dans l'enrobage dur pour capsules ci-dessus.

2. Capsule médicale pour application rectale selon la revendication 1, dans laquelle l'alkylcellulose est choisie dans le groupe comprenant la méthylcellulose, l'éthylcellulose et la propylcellulose.

3. Capsule médicinale pour application rectale selon la revendication 1, dans laquelle l'hydroxy-

alkylcellulose est choisie dans le groupe comprenant l'hydroxyéthylcellulose, l'hydroxypropylcellulose, l'hydroxy-éthyl-hydroxypropylcellulose et l'hydroxy-éthyl-hydroxybutylcellulose.

4. Capsule médicinale pour application rectale selon la revendication 1, dans laquelle l'hydroxyalkyl-alkylcellulose est choisie dans le groupe comprenant l'hydroxyéthylméthylcellulose, l'hydroxyéthyl-éthylcellulose, l'hydroxypropylméthylcellulose, l'hydroxypropyléthylcellulose, l'hydroxybutyl-méthylcellulose, l'hydroxybutyléthylcellulose et l'hydroxyéthylhydroxypropylméthylcellulose.

5. Capsule médicinale pour application rectale selon la revendication 1, dans laquelle le groupe mono-acyle aliphatique est choisi dans le groupe comprenant les groupes acétyle, propionyle, butyryle et valéryle.

6. Capsule médicinale pour application rectale selon la revendication 1 dans laquelle les degrés de substitution par les groupes mono-acyle aliphatiques et par les groupes succinyle acides dans l'ester mixte de l'éther de cellulose sont au moins égaux à 0,05 et 0, 1 respectivement, par unité de glucose.

7. Capsule médicinale pour application rectale selon la revendication 1, dans laquelle l'enrobage dur pour capsules présente une épaisseur de paroi comprise entre 100 et 150 µm.

8 Capsule médicinale pour application rectale selon la revendication 1, dans laquelle l'ingrédient thérapeutiquement actif contenu dans l'enrobage dur pour capsules, se trouve sous la forme d'un médicament liquide dont la pression osmotique est substantiellement supérieure à la pression osmotique du liquide rectal.

9. Capsule médicinale pour application selon la revendication 8, dans laquelle la pression osmotique essentiellement supérieure à la pression osmotique du liquide rectal est comprise entre 3430 et 19600 kPa/kg $H_2O$.

10. Capsule médicinale pour application rectale selon la revendication 8, dans laquelle l'ingrédient thérapeutiquement actif contenu dans l'enrobage dur pour capsules sous la forme d'un médicament liquide, présente un pH compris entre 3 et 4.

**Revendications pour l'Etat Contractant AT**

1. Procédé de fabrication d'une capsule médicinale pour application rectale comprenant:

a) un enrobage dur pour capsules, réalisé en un ester mixte d'un éther de cellulose, choisi dans le groups des alkylcelluloses, hydroxyalkylcelluloses et hydroxyalkylalkylcelluloses, et estérifié par des groupes mono-acyle aliphatiques et par des groupes succinyle acides, et

b) un ingrédient thérapeutiquement actif susceptible d'être absorbé par voie rectale et enrobé dans l'enrobage dur pour capsules ci-dessus,

la capsule dure étant formée par l'ester mixte et puis la capsule formée étant remplie et obturée.

2. Procédé de fabrication selon la revendication 1, dand lequel l'alkylcellulose est choisie dans le groupe comprenant la méthylcellulose, l'éthylcellulose et la propylcellulose.

3. Procédé de fabrication selon revendication 1, dans lequel l'hydroxyalkylcellulose est choisie dans le groupe comprenant l'hydroxyéthylcellulose, l'hydroxypropylcellulose, l'hydroxy-éthylhydroxy-propylcellulose et l'hydroxy-éthyl-hydroxybutylcellulose.

4. Procédé de fabrication selon revendication 1, dans lequel l'hydroxyalkyl-alkylcellulose est choisie dans le groups comprenant l'hydroxyéthylméthylcellulose, l'hydroxyéthyléthylcellulose, l'hydroxypropyl-méthylcellulose, l'hydroxypropyléthylcellulose, l'hydroxybutylméthylcellulose, l'hydroxybutyl-éthylcellulose et l'hydroxyéthylhydroxypropylméthylcellulose.

5. Procédé de fabrication selon revendication 1, dans lequel le groupe mono-acyle aliphatique est choisi dans le groupe comprenant les groupes acétyle, propionyle, butyryle et valéryle.

6. Procédé de fabrication selon revendication 1, dans lequel les degrés de substitution par les groupes mono-acyle aliphatiques et par les groupes succinyle acides dans l'ester mixte de l'éther de cellulose sont au moins égaux à 0,05 et 0,1 respectivement, par unité de glucose.

7. Procédé fabrication selon revendication 1, dans lequel l'enrobage dur pour capsules présente une épaisseur de paroi comprise entre 100 et 150 µm.

8. Procédé fabrication selon la revendication 1, dans lequel l'ingrédient thérapeutiquement actif contenu dans l'enrobage dur pour capsules, se trouve sous la forme d'un médicament liquide dont la pression osmotique est substantiellement supérieure à la pression osmotique du liquide rectal.

9. Procédé de fabrication selon revendication 8, dans lequel la pression osmotique essentiellement supérieure à la pression osmotique du liquide rectal est comprise entre 3430 et 19600 kPa/kg $H_2O$.

10. Procédé de fabrication selon revendication 8, dans lequel l'ingrédient thérapeutiquement actif contenu dans l'enrobage dur pour capsules sous la forme d'un médicament liquide, présent un pH compris entre 3 et 4.

**Patentansprüche für die Vertragsstaaten CH, DE, FR, IT, LI, NL und SE**

1. Eine Medikamentenkapsel für die rektale Anwendung, gekennzeichnet durch

(a) eine harte Kapselhülle, die aus einem gemischten Ester eines Celluloseethers hergestellt ist, der aus der Gruppe, bestehend aus Alkylcellulosen, Hydroxyalkylcellulosen und Hydroxyalkylalkylcellulosen ausgewählt und mit aliphatischen Monoacyl- und sauren Succinylgruppen verestert ist, und

(b) einen therapeutischen Wirkstoff der rektal absorbiert und in der erwähnten harten Kapselhülle eingeschlossen werden kann.

2. Die Medikamentenkapsel für die rektale Anwendung nach Anspruch 1, gekennzeichnet durch die aus der Gruppe Methylcellulose, Ethylcellulose und Propylcellulose ausgewählte Alkylcellulose.

3. Die Medikamentenkapsel zur rektalen Anwendung nach Anspruch 1 gekennzeichnet durch die aus der Gruppe der Hydroxyethylcellulose, Hydroxypropylcellulose, Hydroxyethylhydroxypropylcellulose und Hydroxyethylhydroxybutylcellulose ausgewählte Hydroxyalkylcellulose.

4. Die Medeikamentenkapsel zur rektalen Anwendung nach Anspruch 1, gekennzeichnet durch die aus der Gruppe Hydroxyethylmethylcellulose, Hydroxyethylethylcellulose, Hydroxyethylethylcellulose, Hydroxypropylmethylcellulose, Hydroxypropylmethylcellulose, Hydroxypropylethylcellulose, Hydroxybutylmethylcellulose, Hydroxybutylethylcellulose und Hydroxyethylhydroxypropyl methylcellulose ausgewählte Hydroxyalkylalkylcellulose.

5. Die Medikamentenkapsel zur rektalen Anwendung nach Anspruch 1, gekennzeichnet durch die aus der Gruppe der Acetyl-, Propionyl-, Butyryl und Valerylgruppen ausgewählte Monoacylgruppe.

6. Die Medikamentenkapsel zur rektalen Anwendung nach Anspruch 1, dadurch gekennzeichnet, daß die Substitutionsgrade mit den aliphatischen Mono- acyl- und sauren Succinylgruppen in dem gemischten Ester des Celluloseethers mindestens 0,05 bzw. 0,1 pro Glucoseeinheit sind.

7. Die Medikamentenkapsel für die rektale Anwendung nach Anspruch 1, gekennzeichnet, durch eine Wandstärke der harten Kapselhülle im Bereich von 100 bis 150 µm.

8. Die Medikamentenkapsel für die rektale Anwendung nach Anspruch 1, dadurch gekennzeichnet, daß der in der harten Kapselhülle eingeschlossene Wirkstoff die Form eines flüssigen Arzneimittels hat, das einen osmotischen Druck hat, der wesentlich höher als der osmotische Druck der Rektalflüssigkeit ist.

9. Die Medikamentenkapsel für die rektale Anwendung nach Anspruch 8, dadurch gekennzeichnet, daß der osmotische Druck wesentlich höher als der osmotische Druck der Rektalflüssigkeit ist une im Bereich von 3430 bis 19600 kPa/kg $H_2O$ liegt.

10. Die Medikamentenkapsel für die rektale Anwendung nach Anspruch 8, dadurch gekennzeichnet, daß der in der harten Kapselhülle in Form eines flüssigen Arzneimittels eingeschlossene Wirkstoff einen pH-Wert im Bereich von 3 bis 4 hat.

**Patentansprüche für den Vertragsstaat AT**

1. Verfahren zur Herstellung einer Kapsel für die rektale Anwendung, bestehend aus

(a) einer harten Kapselhülle, die aus einem gemischten Ester eines Celluloseethers besteht, der aus der Gruppe bestehend aus Alkylcellulosen, Hydroxyalkylcellulosen und Hydroxyalkylalkylcellulosen ausgewählt und mit aliphatischen Monoacyl- und sauren Succinylgruppen verestert ist und

(b) einem therapeutischen rektal absorbierbaren Wirkstoff, der in der harten Kapselhülle eingeschlossen ist,

wobei zunächst die feste Kapsel aus dem gemischten Ester geformt und dann die vorgeformte Kapsel gefüllt und geschlossen wird.

2. Verfahren nach Anspruch 1, gekennzeichnet durch die aus der Gruppe Methylcellulose, Ethylcellulose und Propylcellulose ausgewählte Alkylcellulose.

3. Das in Anspruch 1 beanspruchte Verfahren, gekennzeichnet durch die aus der Gruppe Hydroxyethylcellulose, Hydroxypropylcellulose, Hydroxyethylhydroxypropylcellulose und Hydroxyethylhydroxybutylcellulose ausgewählte Hydroxyalkylcellulose.

4. Das nach Anspruch 1 beanspruchte Verfahren, gekennzeichnet durch die aus der Gruppe Hydroxyethylmethylcellulose, Hydroxyethylethylcellulose, Hydroxypropylmethylcellulose, Hydroxypropylmethylcellulose, Hydroxypropylethylcellulose, Hydroxybutylmethylcellulose, Hydroxybutylethylcellulose und Hydroxyethylhydroxypropylmethylcellulose ausgewählte Hydroxyalkylalkylcellulose.

5. Das nach Anspruch 1 beanspruchte Verfahren, gekennzeichnet durch die aus der Gruppe Acetyl-, Propionyl-, Butyryl- und Valerylgruppe ausgewählte aliphatische Monoacylgruppe.

6. Das nach Anspruch 1 beanspruchte Verfahren, dadurch gekennzeichnet, daß die Substitutionsgrade der aliphatischen Monoacyl- und sauren Succinylgruppen in dem gemischten Ester des Celluloseethers pro Glucoseeinheit mindestens 0,05 bzw. 0,1 sind.

7. Das nach Anspruch 1 beanspruchte Verfahren, gekennzeichnet durch die Wandstärke der harten Kapselhülle von 100 bis 150 µm.

8. Das nach Anspruch 1 beanspruchte Verfahren, dadurch gekennzeichnet, daß der in der harten Kapselhülle eingeschlossene therapeutische Wirkstoff in Form eines flüssigen Arzneimittels vorliegt und einen osmotischen Druck hat, der wesentlich höher als der osmotische Druck der Rektalflüssigkeit ist.

9. Das nach Anspruch 8 beansprucht Verfahren, dadurch gekennzeichnet, daß der osmotische Druck wesentlich höher als der osmotische Druck der Rektalflüssigkeit ist une im Bereich von 3430 bis 19600 kPa/kg $H_2O$ liegt.

10. Das nach Anspruch 8 beanspruchte Verfahren, dadurch gekennzeichnet, daß der in der harten Kapselhülle eingeschlossene therapeutische Wirkstoff in Form eines flüssigen Arzneimittels einen pH-Wert hat, der im Bereich von 3 bis 4 liegt.

FIG. 1

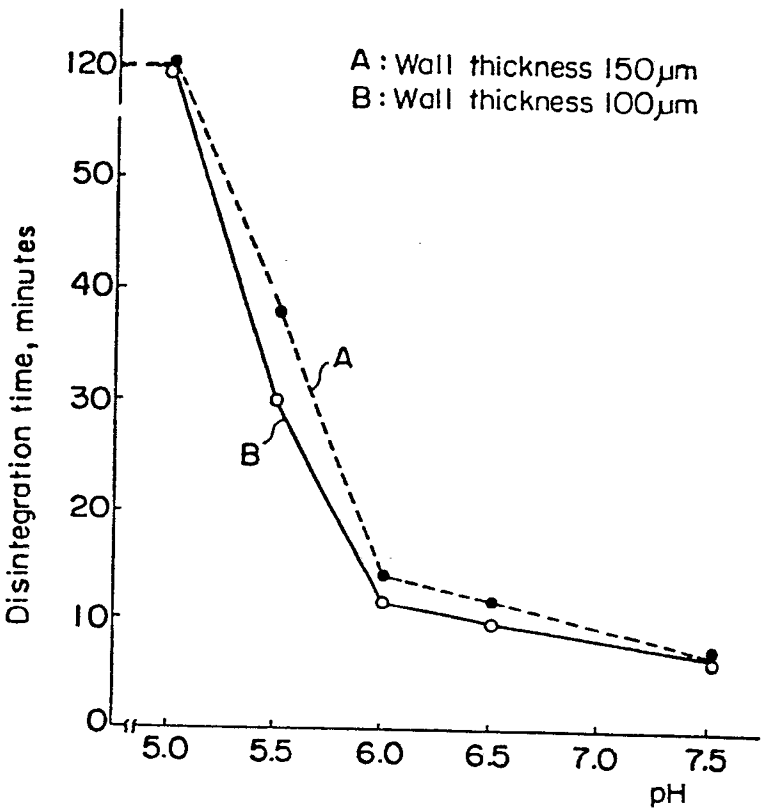
A : Wall thickness 150μm
B : Wall thickness 100μm
Disintegration time, minutes
120
50
40
30
20
10
0
A
B
5.0
5.5
6.0
6.5
7.0
7.5
pH

FIG. 2

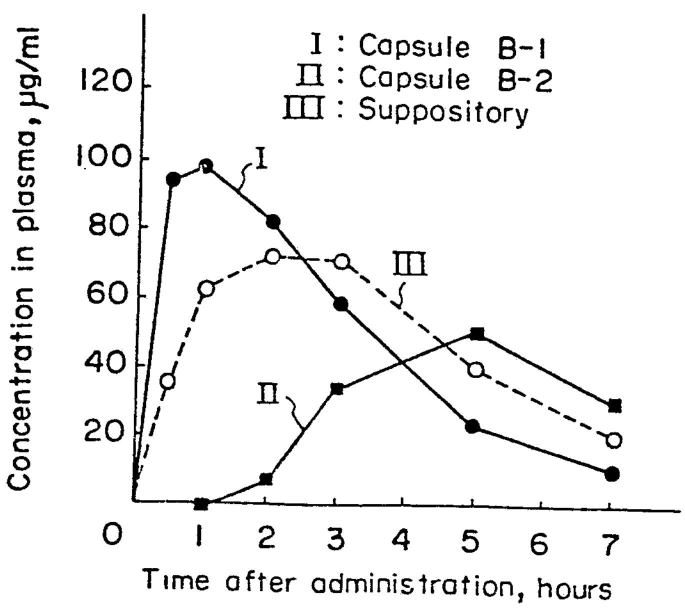
I : Capsule B-1
II : Capsule B-2
III : Suppository
Concentration in plasma, μg/ml
120
100
80
60
40
20
0
I
II
III
1
2
3
4
5
6
7
Time after administration, hours

FIG. 3

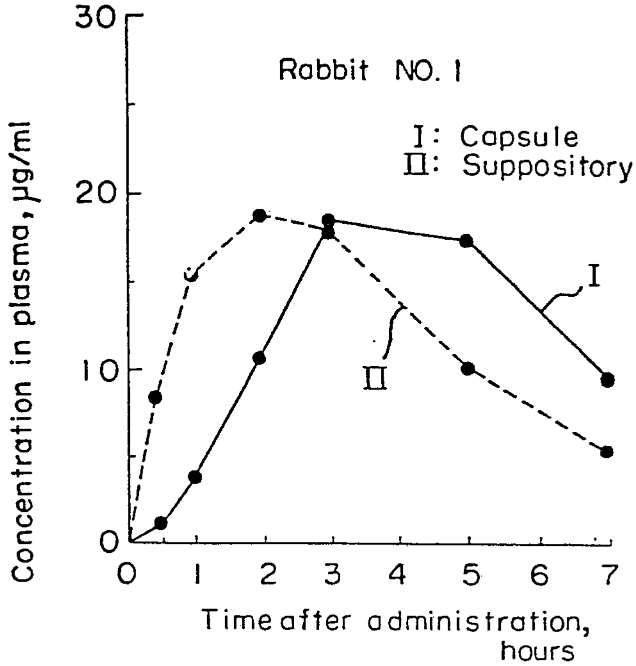

Rabbit NO. 1
I: Capsule
II: Suppository
Concentration in plasma, μg/ml
30
20
10
0
0 1 2 3 4 5 6 7
Time after administration, hours
I
II


FIG. 4

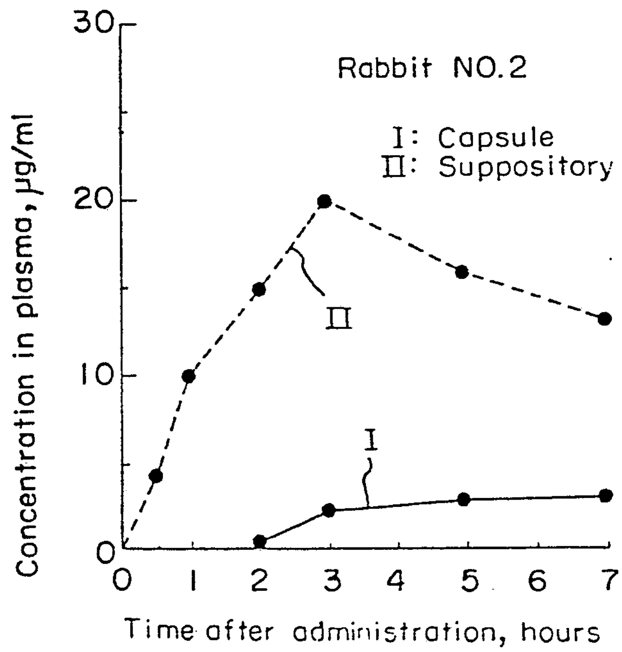

Rabbit NO. 2
I: Capsule
II: Suppository
Concentration in plasma, μg/ml
30
20
10
0
0 1 2 3 4 5 6 7
Time after administration, hours
II
I

FIG.5

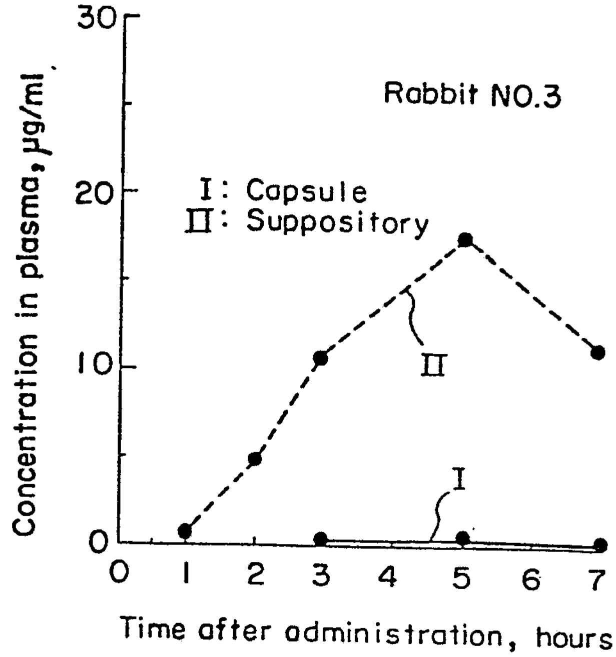
Rabbit NO.3
I : Capsule
II : Suppository
II
I
Concentration in plasma, µg/ml
30
20
10
0
0
1
2
3
4
5
6
7
Time after administration, hours

FIG.6

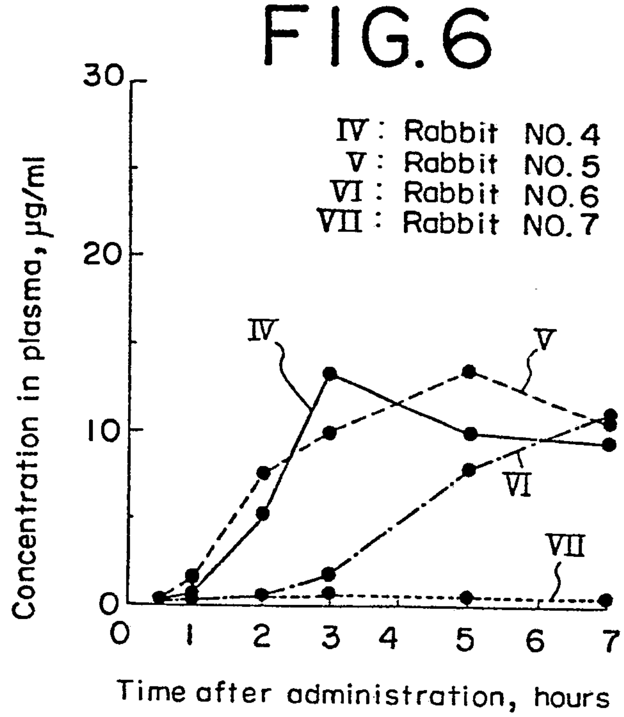
IV : Rabbit NO. 4
V : Rabbit NO. 5
VI : Rabbit NO.6
VII : Rabbit NO. 7
IV
V
VI
VII
Concentration in plasma, µg/ml
30
20
10
0
0
1
2
3
4
5
6
7
Time after administration, hours

FIG. 7

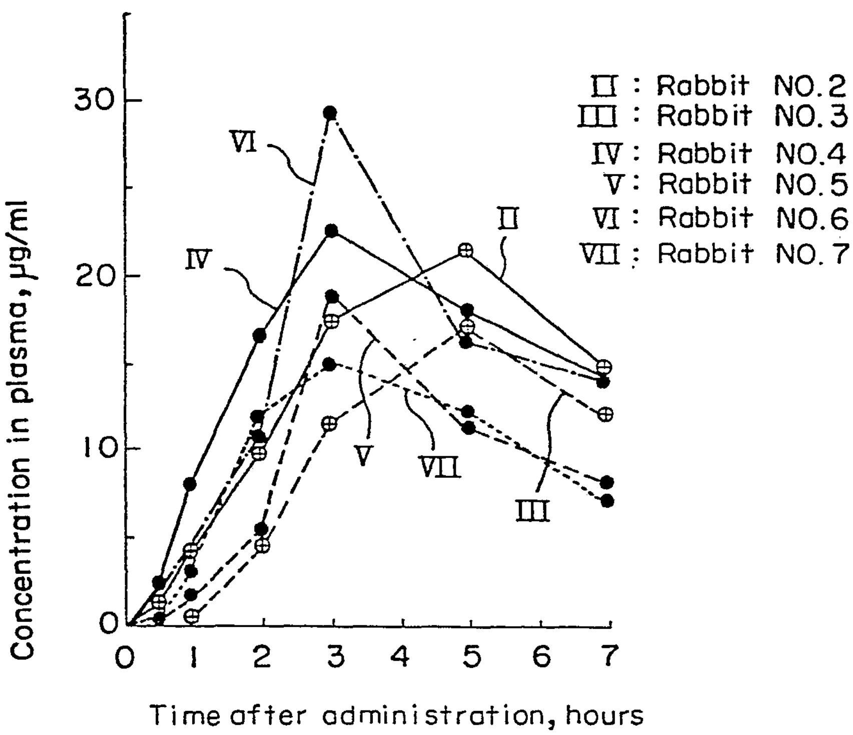
30
20
10
0
Concentration in plasma, μg/ml
0
1
2
3
4
5
6
7
Time after administration, hours
VI
IV
II
V
VII
III
II : Rabbit NO. 2
III : Rabbit NO. 3
IV : Rabbit NO. 4
V : Rabbit NO. 5
VI : Rabbit NO. 6
VII : Rabbit NO. 7